# EUROPEAN PATENT APPLICATION

(11) **EP 4 317 131 A1**
(43) Date of publication of application: **07.02.2024**
(21) Application number: 21935323.2
(22) Date of filing: 15.11.2021
(51) Int. Cl.: C07C 303/44, C07C 309/65, C11C 3/12, C11C 3/10

(54) **METHOD FOR PRODUCING FATTY ACID ALKYL ESTER SULFONATE BY USING COFFEE OIL**

(30) Priority: 29.03.2021 KR 20210040328
(71) Applicant: Bjbiochem Co., Ltd., Yuseong-gu, Daejeon 34054 (KR)
(72) Inventor: JEONG, Gug In, Daejeon 34025 (KR); CHA, Kyung On, Daejeon 34025 (KR); WON, Chae Hoon, Daejeon 34025 (KR)
(74) Representative: AWA Sweden AB
(86) International application number: PCT/KR2021/016619
(87) International publication number: WO 2022/211210

(57) **Abstract**

The present invention relates to a method for producing a fatty acid alkyl ester sulfonate by using a coffee-derived oil. The fatty acid alkyl ester sulfonate produced by using a coffee-derived oil of the present invention had excellent washing ability compared with commercially available LAS. In addition, a detergent containing the fatty acid alkyl ester sulfonate produced by using a a coffee-derived oil showed washing ability equivalent to or higher than that of a detergent containing a fatty acid alkyl ester sulfonate produced by using palm oil, which confirmed that coffee oil can replace palm oil.

## Description

### [Technical Field]

The present invention relates to a method of preparing fatty acid alkyl ester sulfonates using coffee oil.

### [Background Art]

Conventionally, fatty acid methyl esters were mainly synthesized using palm oil or tallow, and fatty acid methyl ester sulfonates (MESs) were synthesized through sulfonation of fatty acid methyl esters. As described above, fatty acid methyl ester sulfonates synthesized using palm or tallow oil have been used as surfactants in a variety of household chemicals such as laundry detergents, hard surface cleaners, and dishwashing agents.

Thereamong, palm oil was once spotlighted as a sustainable oil due to the advantage in that it contains a vegetable ingredient, and has grown as the most generally used plant oil all around the world because it can be produced all year round, is cost-effective, having an oil content per fruit weight 3-fold higher than coconut oil and 10-fold higher than soybean oil and is economically mass-produced. Therefore, palm oil is widely used not only for food, but also as a basic raw material for biodiesel, detergents, cosmetics and the like. However, the repeated burning of rainforests to form palm farms has the adverse effect of destroying the environment. In fact, Indonesia, which is the largest palm oil-producing nation, burns rainforests corresponding to three-quarters of the land area of Korea every year to expand palm farms. Indonesia, which was once called "the lungs of Asia" due to the large area of tropical rainforest therein, is now the third largest greenhouse gas-emitting nation in the world.

The destruction of rainforests due to the expansion of palm oil has created problems associated with animal habitats and climates. In response thereto, the EU discussed a bill to phase out biodiesel made from palm oil, and palm oil-producing nations in Southeast Asia filed a complaint with the WTO. The concept of "palm-free" is used for consumer products that have used palm oil and the possibility of regulation on consumer products using palm is emerging. Algae oil, soybean oil, coconut oil and the like using marine algae are discussed as alternatives to palm oil, but barriers to market expansion are strong due to low price competitiveness compared to palm oil. Therefore, there is an urgent need for novel plant oils that can replace palm oil.

### [Disclosure]

### [Technical Problem]

It is one aspect of the present invention to provide a method of preparing fatty acid alkyl ester sulfonates using oils extracted from coffee beans as well as oils extracted from wasted coffee grounds.

### [Technical Solution]

In accordance with the present invention, the above and other objects can be accomplished by the provision of a method of preparing fatty acid alkyl ester sulfonates using oils extracted from coffee beans as well as oils extracted from wasted coffee grounds.

In accordance with another aspect of the present invention, there is provided fatty acid alkyl ester sulfonate containing a C16 or C18 moiety as a main component, prepared by the method.

In accordance with another aspect of the present invention, there is provided a detergent composition containing the fatty acid alkyl ester sulfonate prepared by the method.

### [Advantageous Effects]

The present invention is capable of providing a method of preparing fatty acid alkyl ester sulfonates using wasted coffee grounds.

The fatty acid alkyl ester sulfonate prepared using the coffee-derived oil of the present invention had superior cleaning capacity compared to commercially available alkylbenzene sulfonate (LAS).

In addition, the detergent containing the fatty acid alkyl ester sulfonate prepared using the coffee-derived oil exhibits cleaning capacity equal to or higher than that of the detergent containing the fatty acid alkyl ester sulfonate prepared using the palm oil, which indicates that the coffee-derived oil can be used as an alternative to palm oil. Meanwhile, among coffee-derived oils, oils extracted from coffee grounds have a composition and quality similar to that of oils extracted from coffee beans and these oils can be obtained at a lower price than the cost of cultivating palm oil, thus being economically advantageous.

### [Description of Drawings]

FIG. 1 shows the cleaning capacity of fatty acid methyl ester sulfonate (MES) to WFK10D, which is a stained cotton cloth;
FIG. 2 shows the cleaning capacity of fatty acid methyl ester sulfonate (MES) to WFK20D, which is a stained blend cloth; and
FIG. 3 shows the cleaning capacity of fatty acid methyl ester sulfonate (MES) to JIS stained cloth which is a daily contaminated cotton cloth.

### [Best Mode]

In one aspect, the present invention is directed to a method of preparing fatty acid alkyl ester sulfonate, the method including a first step of adding a short-chain alcohol and a basic catalyst to an oil extracted from coffee beans or coffee grounds, followed by transesterification to prepare unsaturated fatty acid alkyl ester, a second step of adding hydrogen to the unsaturated fatty acid alkyl ester, followed by hydrogenation to prepare saturated fatty acid alkyl ester, and a third step of mixing the saturated fatty acid alkyl ester with a sulfonating agent to synthesize the fatty acid alkyl ester sulfonate.

### Synthesis of unsaturated fatty acid alkyl ester from coffee-derived oil

In the present invention, the method of preparing fatty acid alkyl ester sulfonate includes a first step of adding a short-chain alcohol and a basic catalyst to an oil extracted from coffee beans or coffee grounds, followed by transesterification to prepare unsaturated fatty acid alkyl ester.

As used herein, the term "transesterification" refers to a reaction that is performed using a short-chain alcohol and a basic catalyst.

The alcohol used in this reaction is a C1-C4 alcohol and may be selected from the group consisting of methanol, ethanol, propanol, and butanol, and the alcohol is added in an amount of 2 to 10 mole times, specifically 4 to 6 mole times compared to coffee-derived oil. That is, the coffee-derived oil and alcohol may be mixed in a molar ratio of 1:2 to 1:10.

In the reaction, the basic catalyst may be selected from the group consisting of sodium hydroxide (NaOH), sodium carbonate (Na₂CO₃), sodium alkoxide, potassium hydroxide (KOH), potassium carbonate (K₂CO₃), and potassium alkoxide.

As used herein, the term "coffee-derived oil" refers to an oil that is extracted from coffee beans or grounds and contains an oil in an amount of 10 to 20% based on the weight of the dried coffee grounds.

In the present invention, the oil extracted from coffee or coffee grounds contains C12-C20 or C14-C20 fatty acids and a C16-C18 unsaturated fatty acid as a main component. The coffee-derived oil contains 0.01 to 1% by weight of a C14 fatty acid, 25 to 30% by weight of a C16 fatty acid, 60 to 70% by weight of a C18 fatty acid, and 1 to 10% by weight of a C20 fatty acid.

The coffee-derived oil of the present invention contains the most amounts of a C16 fatty acid (palmitic acid) and a C18 fatty acid (stearic acid) . In the embodiment of the present invention, the fatty acid methyl ester sulfonates prepared using coffee oils containing C12-C20 fatty acids have excellent cleaning effects as surfactants for household chemical products such as detergents.

In addition, in one embodiment of the present invention, the coffee oil has an advantage of having the composition that is the most similar to palm oil among vegetable oils excluding tallow, which is an animal oil, as shown in Table 1 below, and is the most effective oil to replace palm oil because the optimum carbon distribution of fatty acid alkyl ester sulfonate has C16 palmitic acid and C18 stearic acid. Table 1 shows an alkyl composition of an oil such as coffee-extracted oil, palm oil, palm kernel oil, coconut oil, soya oil, or corn oil. All oils other than palm kernel oil and coconut oil contain unsaturated bonds.

In particular, the coffee oil contains 2 to 4% by weight of a C20 unsaturated bond structure and thus exhibits different physical properties. Specifically, when a powder detergent is prepared using fatty acid methyl ester sulfonate (MES) prepared using coffee oil, the surface strength is not excessively weak and adequate. This is because it contains a small amount of MES prepared from C20 arachidic fatty acids in the molecular structure thereof.

**[Table 1]**

| Alkyl chain | ≤ C14 | C16 | C18 | C20 |
|---|---|---|---|---|
| Coffee-extracted oil | | 26-30 | 60-70 | 2-4 |
| Palm Oil | 2 | 44 | 54 | |
| Tallow | 5 | 32 | 63 | |
| Palm kernel oil | 72 | 8 | 20 | |
| Coconut Oil | 81 | 8 | 11 | |
| Soybean | | 11 | 89 | |
| Corn | | 3 | 97 | |

### Hydrogenation of unsaturated fatty acid alkyl esters

The method for preparing fatty acid alkyl ester sulfonate of the present invention includes a second step of adding hydrogen to the unsaturated fatty acid alkyl ester, followed by hydrogenation to prepare saturated fatty acid alkyl ester.

As used herein, the term "hydrogenation" or "hydrogen addition" generally refers to a type of reduction in which hydrogen is added to an unsaturated bond.

As used herein, the term "hydrogenation" is a method of synthesizing an unsaturated organic compound by adding hydrogen to a saturated organic compound. When hydrogen is added to unsaturated fatty acid, the carbon bonds to hydrogen, so a double bond is converted into a single bond to allow the unsaturated fatty acid to be converted into saturated fatty acid, to increase the melting point and make the tissue rigid. In this way, hydrogenation is used in various ways in order to change the physical properties and characteristics of organic compounds.

As used herein, the term "complete hydrogenation" refers to a reaction in which hydrogen is added to unsaturated fatty acid to convert all double bonds into single bonds.

As used herein, the term "incomplete hydrogenation" refers to a reaction in which hydrogen is added to unsaturated fatty acid to convert some double bonds into single bonds.

The iodine value of the unsaturated fatty acid alkyl ester, specifically the unsaturated fatty acid methyl ester, before hydrogenation (hydrogen addition), is about 95 to 115, and saturated fatty acid alkyl ester having an iodine value of 0.03 to 20 is obtained by the complete or partial hydrogenation. The iodine value may be adjusted by controlling the degree of hydrogenation. Specifically, the iodine value can be lowered to 0.03 through complete hydrogenation, or the iodine value of saturated fatty acid alkyl ester, specifically, saturated fatty acid methyl ester can be lowered to 40 through partial hydrogenation. Within these iodine value ranges, the hydrogenation degree is controlled. Preferably, the iodine value is adjusted to 0.03 to 20.

As used herein, the term "iodine value" refers to the average number of non-aromatic double bonds present in the material.

As the iodine number decreases, the function as a detergent becomes better and control over discoloration during sulfonation becomes easier. When the iodine number is 50 or more, the function as a detergent of the final product is greatly deteriorated, and when the iodine number after partial hydrogenation is 10 to 20, the cleaning capacity is slightly deteriorated, but storage stability during production of liquids is improved.

In the present invention, a hydrogenation catalyst may be used for hydrogenation.

The hydrogenation catalyst is represented by the following Formula 1:

[Formula 1] (NiO)ₐ·(MgO)b·(ZnO)_{c}·(Al₂O₃)_{d}

wherein a, b, c and d represent the content based on the total weight of the catalyst, a is 50 to 80% by weight, b is 1 to 10% by weight, c is 1 to 10% by weight, and d is 15 to 30% by weight. Specifically, the content of nickel oxide is 50 to 80% by weight, the content of magnesium oxide is 1 to 10% by weight, the content of zinc oxide is 1 to 10% by weight, and the content of aluminum oxide is 15 to 30% by weight.

Specifically, in Formula 1, a is 60 to 70% by weight, b is 4 to 8% by weight, c is 2 to 6% by weight, and d is 20 to 26% by weight. Therefore, the content of nickel oxide is 60 to 70% by weight, the content of magnesium oxide is 4 to 8% by weight, the content of zinc oxide is 2 to 6% by weight, and the content of aluminum oxide is 20 to 26% by weight.

In the present invention, 320 ml of a (NiO)₆₄·(MgO)₆·(ZnO)₄·(Al₂O₃)₂₃ four-component catalyst pellet was injected, and unsaturated fatty acid alkyl ester prepared in step 1, specifically, unsaturated fatty acid methyl ester was fed. The hydrogenation was performed to prepare saturated fatty acid alkyl ester, specifically, saturated fatty acid methyl ester having an iodine value of 0.03 to 20.

The order of the reactions of the first and second steps, that is, methylation and hydrogenation, may be changed, and hydrogen was added to an oil extracted from coffee beans or grounds to lower the degree of unsaturation and then a short-chain alcohol and a basic catalyst were added thereto to obtain saturated fatty acid alkyl (methyl) ester.

### Sulfonation

The method for preparing fatty acid alkyl ester sulfonate of the present invention includes a third step of mixing the saturated fatty acid alkyl ester with a sulfonating agent to synthesize the fatty acid alkyl ester sulfonate.

The process of mixing the saturated or partially saturated fatty acid methyl ester with a sulfonating agent to synthesize fatty acid methyl ester sulfonate is shown in the following reaction scheme.

The saturated fatty acid methyl ester has only a single bond between carbon atoms and the partially saturated fatty acid methyl ester contains one or more double bonds.

The sulfonating agent may be at least one selected from the group consisting of chlorosulfonic acid, fuming sulfuric acid, sulfur trioxide, sulfuric acid, and acyl sulfate, and specifically may be sulfur trioxide (SO₃), but is not limited thereto.

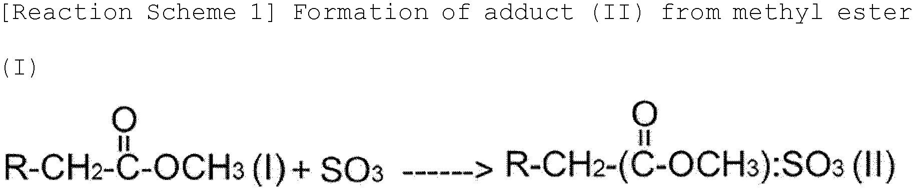

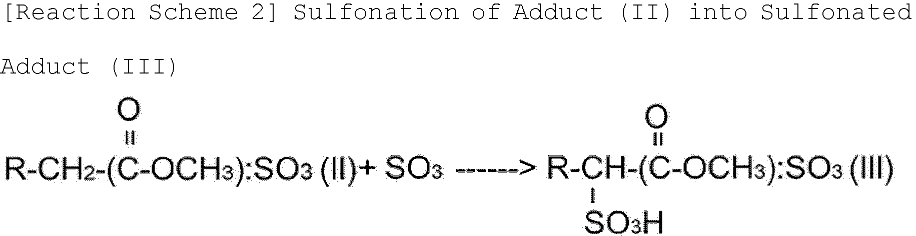

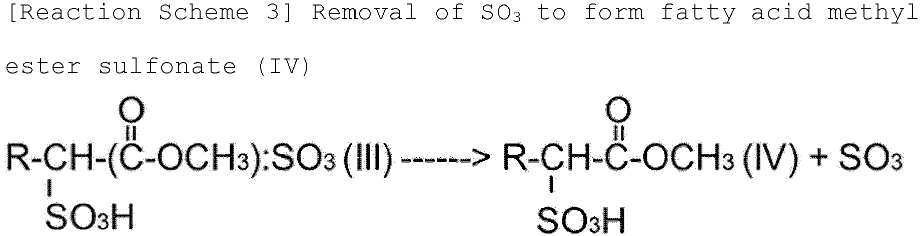

Then, the fatty acid methyl ester sulfonate of Formula (IV) is neutralized with NaOH to prepare fatty acid methyl ester sulfonate.

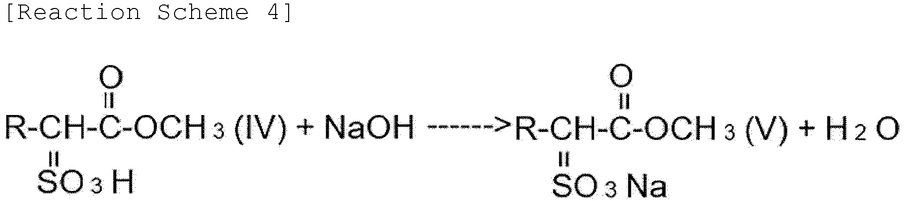

At this time, when the fatty acid methyl ester sulfonate of Formula (IV) is neutralized with NaOH without completely removing SO₃ from the molecular terminal in Reaction Scheme 3, a side reaction occurs as shown in Reaction Scheme 5. The moiety of -COONa is further formed to produce a disalt. At this time, the solubility in water is very high low, resulting in insoluble residues in the final product or performance degradation.

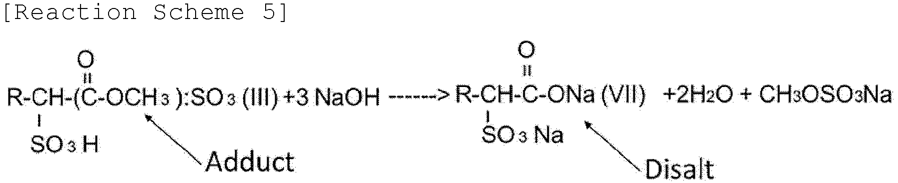

Meanwhile, although the fatty acid methyl ester sulfonate is normally synthesized, hydrolysis occurs, Na is boned to -COOH again to produce -COONa, and a disalt may be also formed through another route different from that shown in Reaction Scheme 6. Therefore, it is important to control various factors such as pH and temperature.

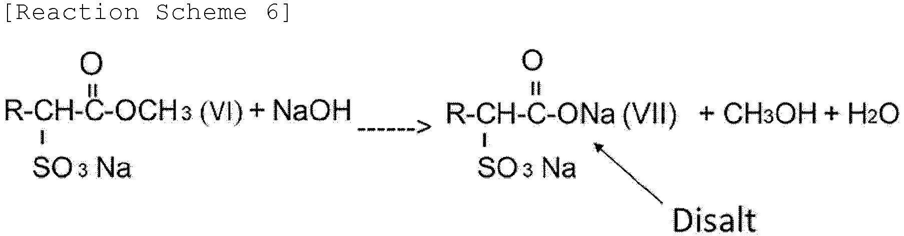

The saturated fatty acid alkyl ester and the sulfonating agent may be mixed in a molar ratio of 1:1 to 1:2, specifically, may be mixed in a molar ratio of 1:1.05.

The prepared fatty acid alkyl ester sulfonate contains a C16 or C18 moiety as a main component and 1 to 10 wt% of a C20 moiety, and is specifically C16 or C18 fatty acid alkyl ester sulfonate.

### Determination of potentials of fatty acid alkyl ester sulfonates in coffee oil

Coffee oil is a plant oil containing greater amounts of C16 and C18 moieties. Since most surfactants commonly used for detergents contain C12 and C14 moieties as main components, MES having each alkyl moiety was prepared using single fatty acids in order to test the applicability of fatty acid alkyl ester sulfonates in coffee oil containing C16 and C18 moieties as main components.

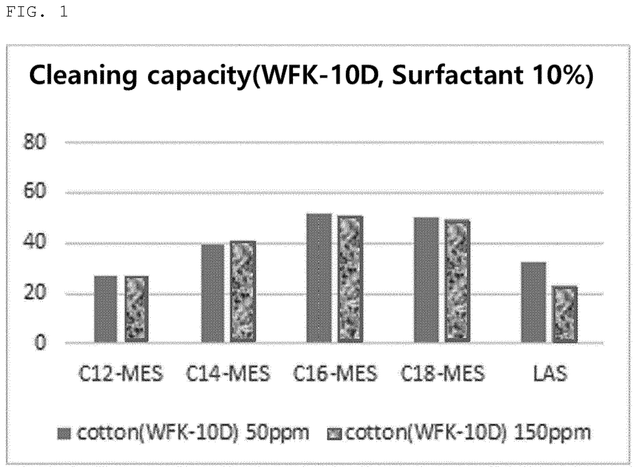

Fatty acid methyl ester sulfonate (MES) was prepared with high purity in accordance with the method described above for each of lauric acid having 12 carbon atoms, myristic acid having 14 carbon atoms, palmitic acid having 16 carbon atoms, and stearic acid having 18 carbon atoms. A detergent containing the MES as a main component was prepared, the cleaning capacity (oil removal ability) thereof was evaluated, and the results are shown in FIGS. 1 to 3.

The cleaning test was performed in a Terg-O-tometer, at 100 RPM and at 20°C for 10 minutes under the concentration conditions of 50 ppm CaCO₃ and 150 ppm CaCO₃ depending on the hardness of water. The stained cloths used for washing were WFK10D (purchased from WFK, Germany, an artificial stained cloth manufacturer), WFK20D (purchased from WFK, Germany, an artificial stained cloth manufacturer), and a JIS stained cloth, a cotton stained cloth (purchased from Japan Laundry Science Association) and measurement was performed using a Colorimeter Nippon Denshoku, D65/10.

The results of the cleaning capacity test of MES prepared for each fatty acid showed that the C16 and C18 fatty acid methyl ester sulfonates (MESs) have the best cleaning capacity and C16 and C18 fatty acid methyl ester sulfonates (MESs) having these carbon distributions exhibit much better cleaning capacity than the most generally used alkylbenzene sulfonates (LASs).

The test showed that C16 and C18 fatty acid methyl ester sulfonates (MESs), which are the characteristic components of coffee oil, were optimal, unlike other surfactants, and are optimal as MES oils.

In addition, the cleaning capacity was compared between the detergent containing the fatty acid methyl ester sulfonate (MES) prepared using the palm oil of Comparative Example 3 and the fatty acid methyl ester sulfonate (MES) prepared using the coffee oil of Example 1. The result showed that there was no significant difference in the cleaning capacity between a detergent containing fatty acid methyl ester sulfonate (MES) prepared using palm oil and a detergent containing fatty acid methyl ester sulfonate (MES) prepared using coffee oil. Therefore, it was found that the detergent prepared from coffee oil was sufficiently competitive like the detergent prepared from palm oil.

In another aspect, the present invention is directed to fatty acid alkyl ester sulfonate containing a C16 or C18 moiety as a main component, prepared by the method.

As used herein, the term "fatty acid alkyl ester sulfonate" is as defined above and is prepared in accordance with the method for preparing fatty acid alkyl ester sulfonate.

In addition, as described above, since the C16 or C18 fatty acid alkyl ester sulfonate exhibits excellent cleaning capacity, fatty acid alkyl ester sulfonate containing 16 carbon atoms or 18 carbon atoms as a main component can be prepared in accordance with the method described above.

In another aspect, the present invention is directed to a detergent composition containing the fatty acid alkyl ester sulfonate prepared by the method.

As used herein, the terms "fatty acid alkyl ester sulfonate" and "method of preparing fatty acid alkyl ester sulfonate" are as described above.

The detergent composition may contain 5 to 20% by weight, specifically 10 to 15% by weight, of fatty acid methyl ester sulfonate.

Examples of the detergent composition include, but are not limited to, hand sanitizers, dishwashing detergents, household detergents, liquid detergents for clothes, cleaners for vegetables and fruits and baby bottle cleaners.

In addition, the fatty acid alkyl ester sulfonate is applicable as a surfactant to a variety of household chemical products. Any household chemical product containing a surfactant may be used without limitation and examples of the household chemical products include wet tissue, deodorants, air fresheners, disinfectants, fungistatic agents, shampoo, conditioners, hair conditioners, foam cleansers, body cleansers, liquids and soaps and the like.

### [Mode for Invention]

Hereinafter, examples of the present invention will be described in detail with reference to the accompanying drawings so that it can be easily implemented by those skilled in the art. However, the prevent invention can be implemented in a variety of different forms and is not limited to embodiments disclosed herein.

### <Experimental Example 1> Composition of fatty acid of oil

The composition of the alkyl group of fatty acids between coffee oil extracted from coffee grounds and other oils was compared. As a result, the coffee oil had the most similar alkyl distribution to palm oil compared to other oils.

**[Table 2]**

| Alkyl chain | ≤ C14 | C16 | C18 | C20 |
|---|---|---|---|---|
| Coffee-extracted oil | | 26-30 | 60-70 | 2-4 |
| Palm oil | 2 | 44 | 54 | |
| Tallow | 5 | 32 | 63 | |
| Palm kernel oil | 72 | 8 | 20 | |
| Coconut Oil | 81 | 8 | 11 | |
| Soybean | | 11 | 89 | |
| Corn | | 3 | 97 | |

### <Experimental Example 2> Preparation of fatty acid methyl ester sulfonate (MES)

Fatty acid methyl ester sulfonates of Example 1 and Comparative Examples 1 to 3 were prepared as follows.

### Example 1. Preparation of fatty acid methyl ester sulfonate (MES) using coffee oil

### 1) Synthesis of unsaturated fatty acid methyl ester

Methanol was added in a molar ratio of 5 to coffee oil and sodium hydroxide (NaOH) was added as a catalyst and unsaturated fatty acid methyl esters having 14 carbon atoms, 16 carbon atoms, and 18 carbon atoms were synthesized through transesterification. The iodine value of the product was measured in accordance with the Wijis method (A.O.C.S Official method). The iodine value of the synthesized unsaturated fatty acid methyl ester was 106.

### 2) Conversion of saturated fatty acid methyl ester

320 ml of a (NiO)₆₄·(MgO)₆·(ZnO)₄·(Al₂O₃)₂₃ 4-component catalyst pellet was added to a stainless tubular reactor with an inner diameter of 24 mm and the temperature of the reactor was increased to 220°C while supplying hydrogen gas at a rate of GHSV 500 hr⁻¹. Then, the temperature was maintained for 10 hours, the temperature was lowered to the reaction temperature, and hydrogenation was initiated while supplying the unsaturated fatty acid methyl ester (iodine value 106) synthesized in 1) of Example 1. The hydrogenation was performed by changing the reaction temperature, reaction pressure, reactant feed rate, and hydrogen supply rate, and the iodine value was decreased to 50 depending on the degree of complete hydrogenation or partial hydrogenation.

The iodine value of the unsaturated fatty acid methyl ester prepared in 1) of Example 1 was 106, and the iodine value of the fatty acid methyl ester synthesized by hydrogenation in method 2) of Example 1 depended on the degree of hydrogenation.

In 2) of Example 1, the unsaturated fatty acid methyl ester having an iodine number of 106 was completely hydrogenated to increase the saturation degree to an iodine number of 1.6, was partially hydrogenated to increase the saturation degree to an iodine number of 14, and was also partially hydrogenated to increase the saturation degree to an iodine number of 50.

**[Table 3]**

| | Process |
|---|---|
| Example 1-A | Unsaturated fatty acid methyl ester having iodine number of 106 was completely hydrogenated using coffee oil to increase the saturation degree to iodine number of 1.6 and produce MES |
| Example 1-B | Unsaturated fatty acid methyl ester having iodine number of 106 was partially hydrogenated using coffee oil to increase the saturation degree to iodine number of 14 and produce MES |
| Example 1-C | Unsaturated fatty acid methyl ester having iodine number of 106 was partially hydrogenated using coffee oil to increase the saturation degree to iodine number of 50 and produce MES |

### 3) Synthesis of fatty acid methyl ester sulfonate (MES)

A fatty acid methyl ester metering injection system and a device capable of vaporizing liquid SO₃, mixing the same with dry air, arbitrarily adjusting the concentration of SO₃ gas and injecting the gas were mounted. Fatty acid methyl ester having an iodine number of 1.6, 14, or 50 synthesized in 2) of Example 1 was heated, completely melted, circulated, and maintained at a constant temperature of 50°C. Then, the unsaturated fatty acid methyl ester maintained at 50°C and 20 v/v% of SO₃ gas were injected at a molar ratio of 1:1.05 into a physical mixer equipped with a temperature gauge and a pressure meter to create microbubbles and were then sprayed into a tubular reactor to prepare emulsified sulfonate. At this time, the injection rate was determined such that the residence time of the total reactants was 70 minutes. The physical mixer used herein was a mixer based on high-speed rotation and the tubular reactor had a temperature gauge inside and a jacket for cooling outside to maintain the reaction and aging temperature at 70°C.

As described above, fatty acid methyl ester sulfonate was synthesized using unsaturated fatty acid methyl ester and sulfur trioxide (SO₃) in accordance with the following Reaction Scheme.

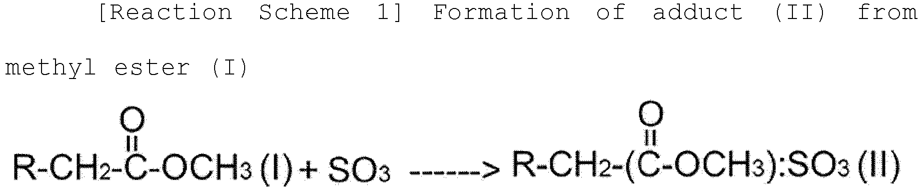

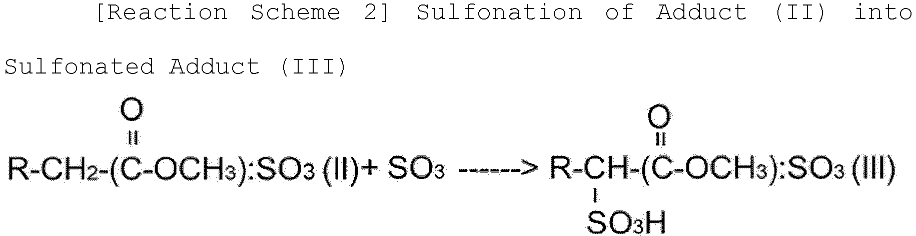

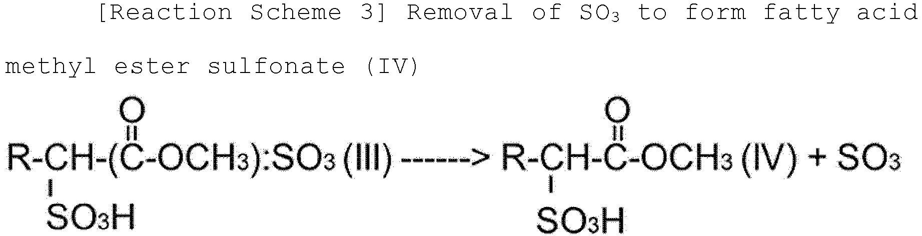

SO₃H is covalently bonded to the α-position as depicted in Formula (III) of [Reaction Scheme 3], is completely removed from the molecular terminal under vacuum, and then is neutralized with NaOH using fatty acid methyl ester sulfonate of Formula (IV) as shown in Reaction Scheme 4 to prepare fatty acid methyl ester sulfonate.

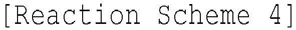

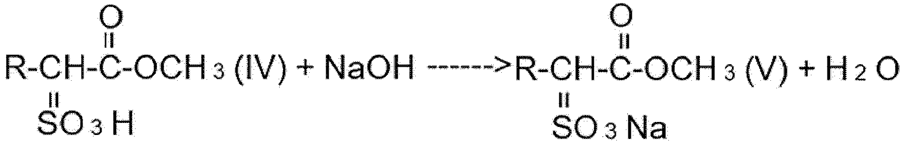

Fatty acid methyl ester sulfonates having 12 carbon atoms, 14 carbon atoms, 16 carbon atoms, and 18 carbon atoms were synthesized using the saturated fatty acid methyl ester prepared in 2) of Example 1 through the steps of Reaction Schemes 1 to 4.

The synthesized fatty acid methyl ester sulfonates were collected from a sampling device installed immediately after the bubbling device by physical mixing and a sampling device at the rear end of the tubular reactor, respectively.

### Comparative Example 1. Preparation of fatty acid methyl ester sulfonate (MES) using coffee oil

### 1) Synthesis of unsaturated fatty acid methyl ester

Unsaturated fatty acid methyl ester having an iodine value of 106 was synthesized in the same manner as in 1) of Example 1.

### 2) Conversion of saturated fatty acid methyl ester

Saturated fatty acid methyl esters having iodine values of 1.6, 14, and 50 were synthesized in the same manner as in 2) of Example 1.

### 3) Synthesis of fatty acid methyl ester sulfonate (MES)

Fatty acid methyl ester sulfonate was synthesized using the saturated fatty acid methyl ester prepared in 2) of Comparative Example 1 in the same manner as in 3) of Example 1, based on the procedures of Reaction Schemes 1 to 3. However, the process of removing SO₃ from the molecular terminal under vacuum was omitted, as shown in Reaction Scheme 4, and neutralization was performed with NaOH as shown in Reaction Scheme 5. As a result, as shown in Reaction Scheme 5 below, -COONa is formed to produce a disalt, and solubility in water is reduced and the feeling of use and performance are deteriorated.

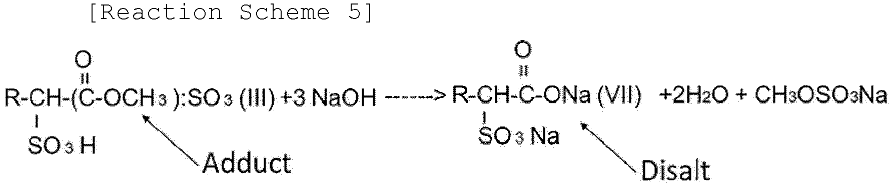

### Comparative Example 2. Preparation of fatty acid methyl ester sulfonate (MES) using coffee oil

### 1) Synthesis of unsaturated fatty acid methyl ester

Unsaturated fatty acid methyl ester having an iodine value of 106 was synthesized in the same manner as in 1) of Example 1.

### 2) Conversion of saturated fatty acid methyl ester

Saturated fatty acid methyl esters having iodine values of 1.6, 14, and 50 were synthesized in the same manner as in 2) of Example 1.

### 3) Synthesis of fatty acid methyl ester sulfonate (MES)

Fatty acid methyl ester sulfonate was synthesized using the saturated fatty acid methyl ester prepared in 2) of Comparative Example 2 in the same manner as 3) of Example 1, based on the procedures of Reaction Schemes 1 to 3. Then, the fatty acid methyl ester sulfonate was hydrolyzed to - COOH and was then neutralized with NaOH as shown in Reaction Scheme 6. As a result, a disalt in the form of -COONa rather than -COOCH₃ was formed as shown in Reaction Scheme 6 below. The moiety of -COONa caused very low solubility and thus was inapplicable as a surfactant to detergent products.

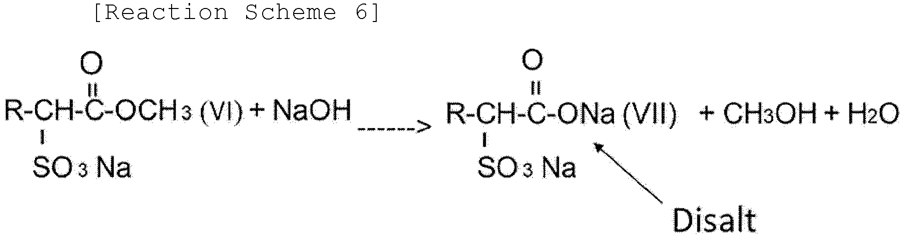

### Comparative Example 3. Preparation of fatty acid methyl ester sulfonate (MES) using palm oil

### 1) Synthesis of unsaturated fatty acid methyl ester

Unsaturated fatty acid methyl ester having an iodine value of 52 was synthesized in the same manner as in 1) of Example 1, except that palm oil was used instead of coffee oil.

### 2) Conversion of saturated fatty acid methyl ester

Saturated fatty acid methyl ester was synthesized using the unsaturated fatty acid methyl ester prepared in 1) of Comparative Example 3, in the same manner as in 2) of Example 1, except that the iodine value thereof was increased to 1.2 by complete hydrogenation.

### 3) Synthesis of fatty acid methyl ester sulfonate (MES)

Fatty acid methyl ester sulfonate was synthesized using the saturated fatty acid methyl ester prepared in 2) of Comparative Example 3 in the same manner as in 3) of Example 1, based on the procedures of Reaction Schemes 1 to 3 and was neutralized with NaOH as depicted in Reaction Scheme 4 to prepare fatty acid methyl ester sulfonate.

### Comparative Example 4. Preparation of fatty acid methyl ester sulfonate (MES) using coconut oil

### 1) Synthesis of unsaturated fatty acid methyl ester

Unsaturated fatty acid methyl ester having an iodine value of 10 was synthesized in the same manner as in 1) of Example 1, except that coconut oil containing C12 and C14 alkyls as main components was used instead of coffee oil.

### 2) Conversion of saturated fatty acid methyl ester

Saturated fatty acid methyl ester was synthesized using the unsaturated fatty acid methyl ester prepared in 1) of Comparative Example 4, in the same manner as in 2) of Example 1, except that the iodine value thereof was increased to 0.8 by complete hydrogenation.

### 3) Synthesis of fatty acid methyl ester sulfonate (MES)

Fatty acid methyl ester sulfonate was synthesized using the saturated fatty acid methyl ester prepared in 2) of Comparative Example 4 in the same manner as in 3) of Example 1, based on the procedures of Reaction Schemes 1 to 3 and was neutralized with NaOH as depicted in Reaction Scheme 4 to prepare fatty acid methyl ester sulfonate.

### Comparative Example 5. Preparation of fatty acid methyl ester sulfonate (MES) using soybean oil

### 1) Synthesis of unsaturated fatty acid methyl ester

Unsaturated fatty acid methyl ester having an iodine value of 132 was synthesized in the same manner as in 1) of Example 1, except that soybean oil containing a C18 alkyl as a main component was used instead of coffee oil.

### 2) Conversion of saturated fatty acid methyl ester

Saturated fatty acid methyl ester was synthesized using the unsaturated fatty acid methyl ester prepared in 1) of Comparative Example 5, in the same manner as in 2) of Example 1, except that the iodine value thereof was increased to 1.9 by complete hydrogenation.

### 3) Synthesis of fatty acid methyl ester sulfonate (MES)

Fatty acid methyl ester sulfonate was synthesized using the saturated fatty acid methyl ester prepared in 2) of Comparative Example 5 in the same manner as in 3) of Example 1, based on the procedures of Reaction Schemes 1 to 3 and was neutralized with NaOH as depicted in Reaction Scheme 4 to prepare fatty acid methyl ester sulfonate.

### <Experimental Example 3> Evaluation of cleaning capacity of fatty acid methyl ester sulfonate (MES)

The cleaning capacity (oil removal ability) was evaluated using the C14, C16 and C18 fatty acid methyl ester sulfonates (MESs) prepared in Example 1. As a control, commercially available LAS (linear alkylbenzene sulfonate) was used.

In addition, since coffee oil contains almost no C12 alkyl, a C12 alkyl was separated from the coffee oil and was used to synthesize C12 fatty acid methyl ester sulfonate (MES) in accordance with the method of Example 1.

The cleaning capacity test was performed in a Terg-O-tometer, at 100 RPM and at 20°C for 10 minutes under the concentration conditions of 50 ppm CaCO₃ and 150 ppm CaCO₃ depending on the hardness of water.

The stained cloths used for cleaning were WFK10D (purchased from WFK, Germany, an artificial stained cloth manufacturer), WFK20D (purchased from WFK, Germany, an artificial stained cloth manufacturer), and a JIS stained cloth, a cotton stained cloth (purchased from Japan Laundry Science Association) and measurement was performed using a Colorimeter Nippon Denshoku, D65/10.

The cleaning capacity of each of C12, C14, C16 and C18 fatty acid methyl ester sulfonates (MESs) is shown in FIGS. 1 to 3.

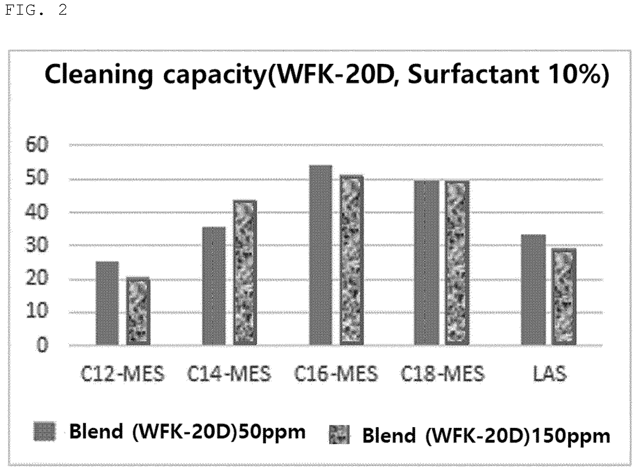

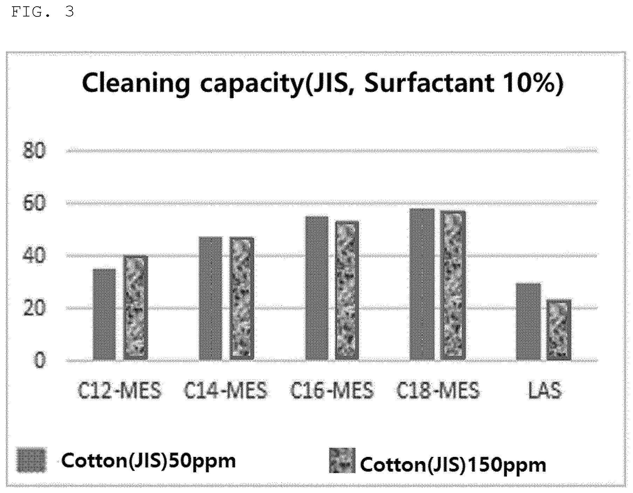

FIG. 1 shows the cleaning capacity of fatty acid methyl ester sulfonate (MES) to WFK10D, which is a stained cotton cloth. FIG. 2 shows the cleaning capacity of fatty acid methyl ester sulfonate (MES) to WFK20D, which is a stained blend cloth. FIG. 3 shows the cleaning capacity of fatty acid methyl ester sulfonate (MES) to JIS stained cloth which is a daily contaminated cotton cloth.

As shown in FIGS. 1 to 3, the results of the cleaning capacity test showed that the C16 and C18 fatty acid methyl ester sulfonates (MES) have the best cleaning capacity and are much better than the commercially available LAS.

### <Experimental Example 4> Evaluation of cleaning capacity of fatty acid methyl ester sulfonate (MES)

The cleaning capacity (oil removal capacity) was evaluated using fatty acid methyl ester sulfonates (MES) prepared using Example 1-A, in which the saturation degree of unsaturated fatty acid methyl ester having an iodine number of 106 was increased to an iodine number of 1.6 by complete hydrogenation, Example 1-B, in which the saturation degree of unsaturated fatty acid methyl ester having an iodine number of 106 was increased to an iodine number of 14 by partial hydrogenation, and Example 1-C, in which the saturation degree of unsaturated fatty acid methyl ester having an iodine number of 106 was increased to an iodine number of 50 by partial hydrogenation, and fatty acid methyl ester sulfonates (MES) prepared in Comparative Examples 3 to 5.

**[Table 4]**

| | Stain removal (%) | | | | | |
|---|---|---|---|---|---|---|
| | 50 ppm | | | 150 ppm | | |
| | WFK-10D (cotton ) | WFK-20D (blended) | JIS (cotto n) | WFK-10D (cotton) | WFK-20D (blended ) | JIS (cotton) |
| Example 1-A | 75 | 80 | 83 | 74 | 78 | 79 |
| Example 1-B | 70 | 74 | 79 | 68 | 71 | 75 |
| Example 1-C | 54 | 70 | 70 | 66 | 66 | 68 |
| Comparati ve Example 3 | 77 | 80 | 83 | 74 | 77 | 79 |
| Comparati ve Example 4 | 63 | 68 | 76 | 61 | 73 | 72 |
| Comparati ve Example 5 | 71 | 74 | 75 | 70 | 71 | 70 |

In Example 1-A, MES was prepared by completely hydrogenating unsaturated fatty acid methyl ester having an iodine number of 106 using coffee-derived oil to increase the saturation degree to an iodine number of 1.6. In Example 1-B, MES was prepared by partially hydrogenating unsaturated fatty acid methyl ester having an iodine number of 106 using coffee-derived oil to increase the saturation degree to an iodine number of 14. In Example 1-C, MES was prepared by partially hydrogenating unsaturated fatty acid methyl ester having an iodine number of 106 using coffee-derived oil to increase the saturation degree to an iodine number of 50. In Comparative Example 3, MES was prepared by completely hydrogenating unsaturated fatty acid methyl ester having an iodine number of 52 using palm oil to increase the saturation degree to an iodine number of 1.2. In Comparative Example 4, MES was prepared by completely hydrogenating unsaturated fatty acid methyl ester having an iodine number of 10 using coconut oil containing C12 and C14 alkyls as main components to increase the saturation degree to an iodine number of 0.8. In Comparative Example 5, MES was prepared by completely hydrogenating unsaturated fatty acid methyl ester having an iodine number of 132 using soybean oil containing a C18 alkyl as a main component to increase the saturation degree to an iodine number of 1.9.

The cleaning capacity test was performed in the same manner as in Experimental Example 3. The result showed that the coffee oil of Example 1-A exhibited sufficient washing performance to replace the palm oil of Comparative Example 3. When an unsaturated bond having an iodine number of 50 or more was present as in Examples 1-3, the cleaning performance was slightly lowered. However, the cleaning capacity when the C18 ratio was extremely high as in Comparative Example 5 was lower than that when an oil containing a mixture of C16 and C18 was used as in Example 1-A or Comparative Example 3.

In addition, Comparative Example 4 in which the coconut oil contained C12 and C14 alkyls as main components exhibited lower cleaning capacity than Example 1-A or Comparative Example 3 in which a mixed oil of C16 and C18 alkyls was used.

Therefore, saturated fatty acid alkyl esters having an iodine number of 0.3 to 20 were prepared using an oil containing C16 and C18 as main components and the MESs of Examples 1-A and 1-B, and Comparative Example 3 prepared using the same were found to exhibit the best cleaning capacity.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A method of preparing fatty acid alkyl ester sulfonate, the method comprising:
a first step of adding a short-chain alcohol and a basic catalyst to an oil extracted from coffee beans or coffee grounds, followed by transesterification to prepare unsaturated fatty acid alkyl ester;
a second step of adding hydrogen to the unsaturated fatty acid alkyl ester, followed by complete or partial hydrogenation to prepare saturated fatty acid alkyl ester; and
a third step of mixing the saturated fatty acid alkyl ester with a sulfonating agent to synthesize the fatty acid alkyl ester sulfonate.

2. The method according to claim 1, wherein the oil comprises a C14-C20 unsaturated fatty acid as a main ingredient.

3. The method according to claim 1, wherein the oil comprises 0.1 to 10% by weight of a C14 fatty acid, 25 to 30% by weight of a C16 fatty acid, 60 to 70% by weight of a C18 fatty acid, and 1 to 10% by weight of a C20 fatty acid.

4. The method according to claim 1, wherein the second step comprises preparing saturated fatty acid alkyl ester having an iodine value of 0.03 to 20 by complete or partial hydrogenation.

5. The method according to claim 1, wherein the alcohol is a C1-C4 short-chain alcohol.

6. The method according to claim 1, wherein the basic catalyst in the first step is selected from the group consisting of sodium hydroxide (NaOH), sodium carbonate (Na₂CO₃), sodium alkoxide, potassium hydroxide (KOH), potassium carbonate (K₂CO₃), and potassium alkoxide.

7. The method according to claim 1, wherein the saturated fatty acid alkyl ester and the sulfonating agent are mixed in a molar ratio of 1:1 to 1:2.

8. The method according to claim 1, wherein the fatty acid alkyl ester sulfonate comprises a C16 or C18 moiety as a main ingredient and 1 to 10 wt% of a C20 moiety.

9. The method according to claim 1, wherein the order of the first and second steps is changed and the saturated fatty acid alkyl ester is prepared by adding hydrogen to the oil extracted from coffee beans or grounds to lower the degree of unsaturation, and then further adding the short-chain alcohol and the basic catalyst thereto.

10. Fatty acid alkyl ester sulfonate comprising a C16 or C18 moiety as a main component, prepared by the method according to any one of claims 1 to 8.

11. A detergent composition comprising the fatty acid alkyl ester sulfonate prepared by the method according to any one of claims 1 to 9.
